# EUROPEAN PATENT SPECIFICATION

(11) **EP 2 170 837 B1**
(45) Date of publication and mention of the grant of the patent: **02.11.2011**
(21) Application number: 08771774.0
(22) Date of filing: 24.06.2008
(51) Int. Cl.: C07D 239/36, C07D 239/47

(54) **PROCESS FOR PREPARING 2-(3-{6-[2-(2,4-DICHLOROPHENYL)-ETHYLAMINO]-2-METHOXYPYRIMIDIN-4-YL)-PHENYL)-2-METHYLPROPIONIC ACID**
VERFAHREN ZUR HERSTELLUNG VON 2-(3-{6-[2-(2,4-DICHLORPHENYL)ETHYLAMINO]-2-METHOXYPYRIMIDIN-4-YL}PHENYL)-2-METHYLPROPIONSÄURE
PROCÉDÉ DE PRÉPARATION DE L'ACIDE 2-(3-{6-(2-(2,4-DICHLOROPHÉNYL)-ÉTHYLAMINO)-2-MÉTHOXYPYRIMIDIN-4-YL}-PHÉNYL)- 2-MÉTHYLPROPIONIQUE

(30) Priority: 29.06.2007 US 947256 P
(43) Date of publication of application: 07.04.2010
(73) Proprietor: SANOFI, 75013 Paris (FR)
(72) Inventor: HUANG, Bao-Guo, Bridgewater, New Jersey 08807 (US); HANNA, Reda G., Bridgewater, New Jersey 08807 (US); SECORD, Elizabeth, Bridgewater, New Jersey 08807 (US); POWERS, Matthew R., Bridgewater, New Jersey 088007 (US)
(74) Representative: Tepfenhárt, Dóra Andrea
(86) International application number: PCT/US2008/067963
(87) International publication number: WO 2009/006086

(56) References cited:
- WO-A-2006/044732
- WO-A-2007/047378

## Description

### Field of the Invention

This invention is directed to a process for preparing 2-(3-{6-[2-(2,4-dichlorophenyl)-ethylamino]-2-methoxypyrimidin-4-yl}-phenyl)-2-methylpropionic acid.

### Background of the Invention

Patent application WO 2006044732 (hereinafter the '732 application) discloses pyrimidines of Formula (I), 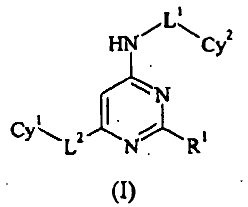 wherein R¹, L¹, L², Cy¹ and Cy² are as defined therein, their preparation, pharmaceutical compositions containing these compounds, and their pharmaceutical use in the treatment of disease states capable of being modulated by the inhibition of the prostaglandin D2 receptor, such as asthma [Arimura A, Yasui K, Kishino J, Asanuma F, Hasegawa H, Kakudo S, Ohtani M, Arita H, Prevention of allergic inflammation by a novel prostaglandin receptor antagonist, S-5751, J Pharmacol Exp Ther. 298(2), 411-9, 2001], allergic rhinitis [Jones, T. R., Savoie, C., Robichaud, A., Sturino, C., Scheigetz, J., Lachance, N., Roy, B., Boyd, M., Abraham, W., Studies with a DP receptor antagonist in sheep and guinea pig models of allergic rhinitis, Am. J Resp: Crit. Care Med. 167, A218, 2003; and Arimura A, Yasui K, Kishino J, Asanuma F, Hasegawa H, Kakudo S, Ohtani M, Arits.H, Prevention of allergic inflammation by a novel prostaglandin receptor antagonist, S-5751.J Pharmacol. Exp. Ther. 298(2), 411-9, 2001], and allergic conjunctivitis and allergic dermatitis [Arimura A, Yasui K, Kishino J, Asanuma F, Hasegawa H, Kakudo S, Ohtani M, Arita H, Prevention of allergic inflammation by a novel prostaglandin receptor antagonist, S-5751. J. Pharmacol. Exp. Ther. 298(2), 411-9, 2001; and Torisu K, Kobayashi K, Iwahashi M, Nakai Y, Onoda T, Nagase T, Sugimoto I, Okada Y, Matsumoto R, Nanbu F, Ohuchida S, Nakai H, Toda M, Discovery of a new class of potent, selective, and orally active prostaglandin D2 receptor antagonists, Bioorg., & Med. Chem. 12, 5361-5378, 2004].

The '732 application discloses a process for preparing pyrimidines of Formula (I) wherein L² is a bond, by reacting a corresponding compound of Formula (V), wherein X² is a halogen, preferably chlorine, or a triflate group, with a boronic acid of Formula (VI), or a boronic acid pinacol ester of formula (XVII) in the presence of cesium carbonate and a complex metal catalyst such as tetrakis(triplienylphosphine)palladium (0) or *pddf*.

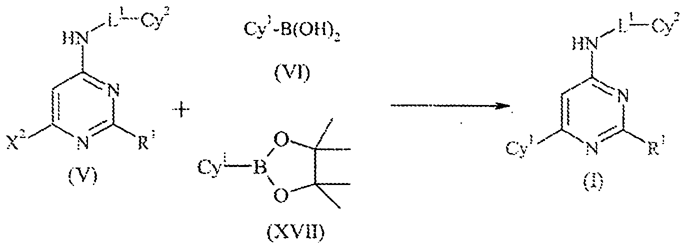

The '732 application further discloses that the compound of Formula (V) wherein X² is chlorine may be prepared by coupling a corresponding dichloropyrimidme of Formula (X) with a corresponding amine of Formula (IV), in the presence of a suitable base, such as sodium bicarbonate.

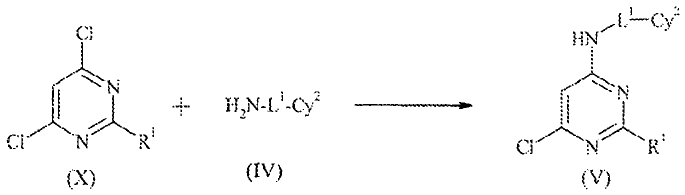

The 732 application more particularly disposes 2-(3-{6-[2-(2,4-dichlorophenyl)-ethylamino]-2-methoxyprimidin-4-yl}-phenyl)-2-methylpropionic acid of formula (A), 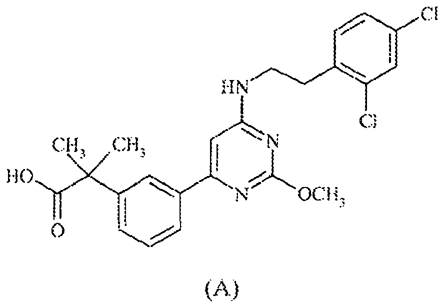 and its preparation as shown in Scheme I.

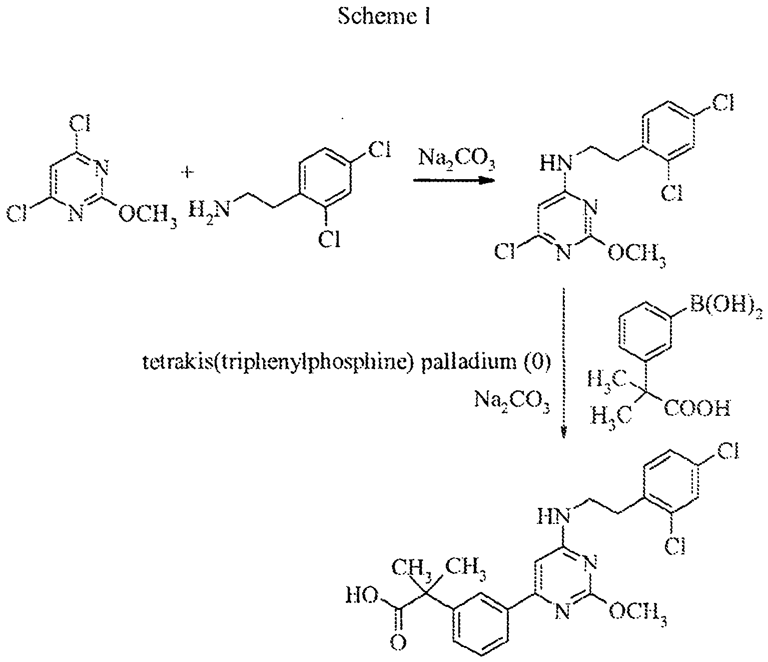

The '732 application also discloses an alternative process for preparing pyrimidines of Formula (I) by reacting a corresponding compound of Formula (III), wherein X¹ is a halogen, particularly chlorine, or a triflate group, with a corresponding amine of Formula (IV), in the a suitable base, such as sodium bicarbonate, in an inert solvent, such as 1-methyl-2-pyrrolidinone, and at a temperature at about 160°C.

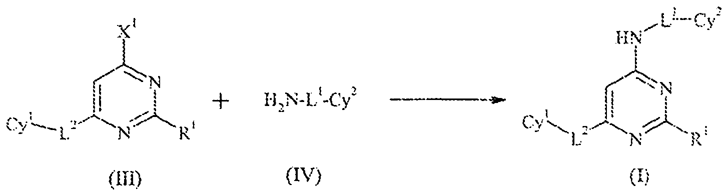

More particularly, Example 43(b) of the '732 application exemplifies the synthesis of [2-methoxy-6-(3-methoxy-phenyl)-pyrimidin-4-yl]-[2-(2-nitro-phenyl)-ethyl]-amine by such alternative process, has shown in Scheme II below:

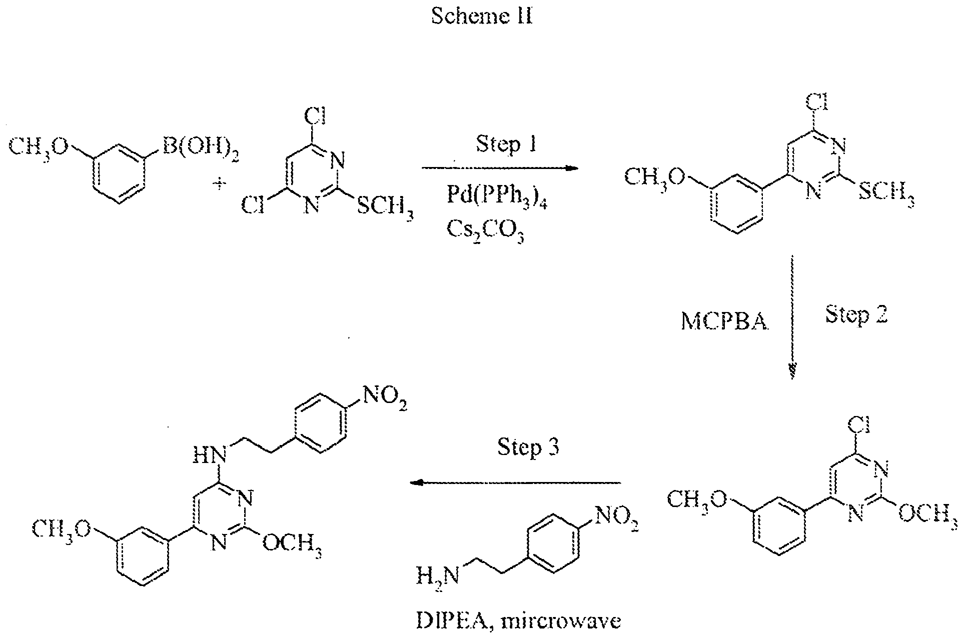

Patent application WO 2007047378 (hereinafter '378 application) also specifically discloses dihydrogen phosphate salt of 2-(3-{6-[2-(2,4-dichlorophenyl)-ethylamino]-2-methoxypyrimidin-4-yl}-phenyl)-2-methylpropionic acid, and its preparation. However, the dihydrogen sail prepared in THF according to the procedures described therein contains about 1400-1600 ppm residual THF, which exceeds the limit of International Conference on Harmonization (ICH) guideline of 700 ppm. Furthermore, recrystallization from acetone is not practical for large scale preparation due to the low solubility.

A process for the preparation of a pharmaceutical active ingredient used in a medicament on an industrial scale has to fulfill various requirements. The process and the obtained product have so be in line with the regulatory requirements and have to be reproducible and validated. In particular, regulatory authorities stipulate a precise degree of purity of the obtained drug substance and an acceptable level (e.g. single digit ppm) for heavy metals such as Pd or Ni. On the other hand, a process performed on an industrial scale for preparing a marketed product should of course be as simple, cost and labor effective as possible. If possible, it should thus avoid the use of expensive starting materials, physiologically unacceptable toxic materials, difficult technical operations, long reaction time, or multiple procedural steps.

2-(3-{6-[2-(2,4-Dichloro-phenyl)-ethylamino]-2-methoxy-pyrimidin-4-yl}-phenyl)-2-methyl-propionic acid prepared by following the procedures described in the '732 application requires chromatographic purification, which is not desirable. In addition, it is also not desirable to remove the residual Palladium catalyst in the last step of the process.

Furthermore, the procedures described in Example 43(b) of the '732 application utilize a high amount (5 mol%) of unstable catalyst, palladium tetrakis(triphenyl-phosphine). In addition, the intermediate and product formed therein also need to be purified by chromatography. Some steps in the process also require long reaction time or use of microwave equipment, which lead to a very low time-space yield and a technical difficulty tor largo scale operation.

Thus, there is a need for a simpler and improved process for manufacturing the compound of formula A. The present invention satisfies this need by providing such a process.

### Summary of the Invention

The present invention is directed to a process for preparing 2-(3-{6-[2-(2,4-dichlorophenyl)-ethylamino]-2-methoxypyrimidin-4-yl}-phenyl)-2-methylpropionic acid of formula (A), 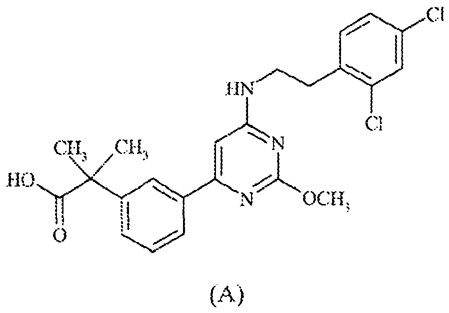 comprising:
(1) coupling 4,6-dichloro-2-methoxypyrimidine with 3-(1-carboxy-1-methyl-ethyl)-phenyl boronic acid in the presence of a suitable Palladium catalyst to provide 2-[3-(6-chloro-2-hydroxy-pyrirnidin-4-yl)-phenyl]-2-methylproplonic acid,
(2) coupling 2-[3-(6-chloro-2-hydroxy-pyrimidin-4-yl)-phenyl]-2-methyl-propionic acid with 2,4-dichlorophenethylamine to provide 2-(3-{6-[2-(2,4-dichlorophenyl)-ethylamino]-2-methoxy-pyrimidin-4-yl}-phenyl)-2-methylpropionic acid.

Another aspect of the invention is the compound 2-[3-(6-chloro-2-hydroxypyrimidin-4-yl)-phenyl]-2-methylpropionic acid, or a salt thereof.

The present invention is more fully discussed with the aid of the following figures and detailed description below.

### Brief Description of the Drawings

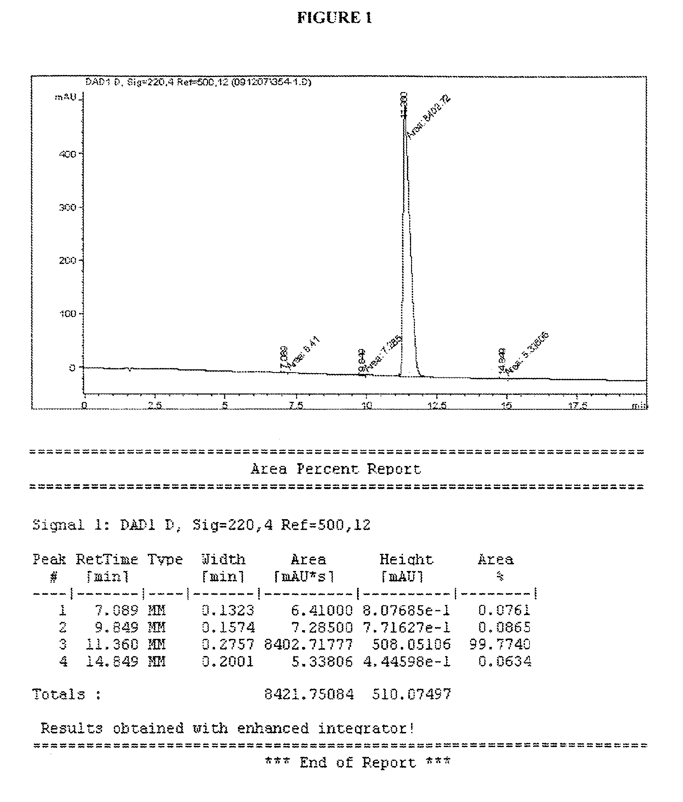

FIGURE 1 is an HPLC spectrum of 2-(3-{6-[2-(2,4-dichloro-phenyl)-ethylamino]-2-methoxy-pyrimidin-4-yl}-phenyl)-2-methyl-propionic acid prepared by the process of the invention.

### Detailed Description of the Invention

The present invention will be better appreciated by reference to the following detailed description.

### Definitions and Abbreviations

As used above, and throughout the description of the invention, the following abbreviations, unless otherwise indicated, shall be understood to have the following meanings: .
- DME: 1,2-dimethoxyethane
- g: gram
- h: hour
- HCl: Hydrochloric acid
- K₂CO₃: Potassium carbonate
- Na₂CO₃: Sodium carbonate
- Cs₂CO₃: Cesium carbonate
- K₃PO₄: Potassium Phosphate
- Na₃PO₄: Sodium Phosphate.
- NaOH: Sodium hydroxide
- KOH: Potassium hydroxide
- mg: milligram
- min: minute
- mL: milliliter
- n-BuOAc: n-butyl acetate
- *pddf*: 1,1'-Bis(diphenylphosphino)ferrocene-palladium(II)dichloride dichloromethane complex
- Pd(OAc)₂: palladium (U) acetate
- Pd(PPh₃)₄: tetrakis(briphenylphosphine)palladium (0)
- PPh₃: triphenylphosphine
- ppm: parts per million
- TMT: trithiocyanuric acid
- THF: tetrahydrofuran
- ~: approximately

As used abode, and throughout the description of the invention, the following terms, unless otherwise indicated, shall be understood to have the following meanings.

"Palladium catalyst" includes, for example, Pd, PdCl₂, Pd(OAc)₂, or *pddf.*

"Base" includes inorganic base, for example, Na₂CO₃, K₂CO₃, C_{S2}CO₃, K₃PO₄, KOH or NaOH, more particularly K₂CO₃, and organic base, for example, triethylamine or diisopropyl ethylamine.

"Solvent system" include organic solvent, for example, alcohol, ethyl acetate, THF, DME, toluene, or a mixture of water soluble organic solvent with water, for example, a mixture of DIVE and water, THF and water, or alcohol and water, more particularly a mixture of DME and water.

### Particular Embodiments of the Invention

In a particular embodiment of the invention, the first step coupling reaction is carried out in the presence of Pd, PdCl₂, Pd(PPh₃)₄, *pddf,* or Pd(OAc)₂; more particularly Pd(OAc)₂.

In another particular embodiment of the invention, the first step coupling reaction is carried out in the presence of Pd(OAc)₂ and PPh₃.

In another particular embodiment of the invention, the first step coupling reaction is carried out in the presence of 0.01 mol% to 0.5 mol% of Pd(OAc)₂ and 0.02 mol% to 1.0 mol% PPh₃, more particularly 0.1 mol% of Pd(OAc)₂ and 0.2 mol% PPh₃.

In another particular embodiment of the invention, the first step coupling reaction is carried out by adding 3-(1-carboxy-1-methyl-ethyl)-phenyl boronic acid to 4,6-dichloro-2-methoxypyrimidine slowly over a period of time, preferably 2-6 hrs, thus, significantly reducing the over reaction byproduct and thermal decomposition of boronic acid.

In another particular embodiment of the invention, the first step coupling reaction is carried out in the presence of a base.

In another particular embodiment of the invention, the first step coupling reaction is carried out in the presence of a base, wherein the amount of the base is in the range of 2-4 equivalents, more particularly, 3.0 equivalents.

In another particular embodiment of the invention, the first step coupling reaction is carried out using 1.0-2.0 equivalents of 4,6-dichloro-2-methoxypyrimidne, more particularly 1.2' equivalents.

In another particular embodiment of the invention, the first step coupling reaction is carried out in the presence of a solvent system.

In another particular-embodiment of the invention, the first step coupling product, 2-[3-(6-chloro-2-hydroxy-pyrirnidin-4-yl)-phenyl]-2-methylpropionic acid, is purified by:
removing excess 4,6-dichloro-2-methoxypyrimidne by a phase separation, adjusting pH of the phase containing 2-[3-(6-chloro-2-hydroxy-pyrinfdin-4-yl)-phenyl]-2-niethytpropionic acid to 6.5 to 7.5, more particularly 7.2, and extracting the phase containing 2-[3-(6-chloro-2-hydroxy-pyrimidin-4-yl)-phenyl]-2-methylpropionic acid with a water-immiscible organic solvent, for example, n-butyl acetate, ethyl acetate or toluene.

In another particular embodiment of the invention, wherein the Palladium catalyst is removed from 2-[3-(6-chloro-2-chydroxy-pyrimidin-4-yl)-phenyt]-2-methyl-propionic acid prior to the second step, for example, by treating 2-[3-(6-chloro-2-hydroxy-pyrimidin-4-yl)-phenyl]-2-methyl-propionic acid with TMT and charcoal, in an organic solvent, such as n-butyl acetate at 50-70°C, particularly, at 70°C.

In another particular embodiment of the invention, the first step product, 2-[3-(6-chloro-2-hydroxy-pyrimidin-4-yl)-phenyl]-2-methyl-propionic acid, is used directly in the second step as a solution.

In another particular embodiment of the invention, the second step is carried out in water.

In another particular embodiment of the invention, the second step product, 2-(3-{6-[2-(2,4-dichlorophenyl)-ethylamino]-2-methoxypyrimidin-4-yl}-phenyl)-2-methylpropiotuic acid, is purified by recrystallization from an organic solvent and water, for example, THF/water or DME/water, more particularly, DME/water.

In another particular embodiment of the invention, the compound of formula (A) is converted into dihydrogen phosphate salt thereof in methanol.

The present invention offers an improved process for preparing 2-(3-{6-[2-(2,4-dichlorophenyl)-ethylamino]-2-methoxypyrimidin-4-yl}-phenyl)-2-methylpropionic acid in high yields arid high purities yet requiring no expensive and time consuming column chromatographic purification as used in the `732 application. The present process uses a less amount of and more stabile catalyst. A simpler phase separation is used to separate the step-1 I product, from excess reagents, and the product purification is achieved by a pH adjustment. The residual Pd removal is also reduced to the desired level (5 ppm) in step-1, thus avoiding handling the problem in the final stage since the final product is less soluble in common organic solvents, making it harder to operate. Furthermore, the step-1 product is concatenated into step-2 reaction without isolation. Overall, the present invention provides a much more efficient process for preparing the desired product in a high purity (>99% pure by HPLC) and high time-space yield. The salt formation carried out in methanol instead of THF as disclosed in the '378 application also eliminates the residual solvent issue.

### Examples

The present invention may be better understood by reference to the following non-limiting Examples, which are exemplary of the invention.

### Example 1:

### 2-(3-{6-[2-(2,4-Dichlorophenyl)-ethylamino]-2-methoxypyrimidin-4-yl}-phenyl)-2-methypropionic acid

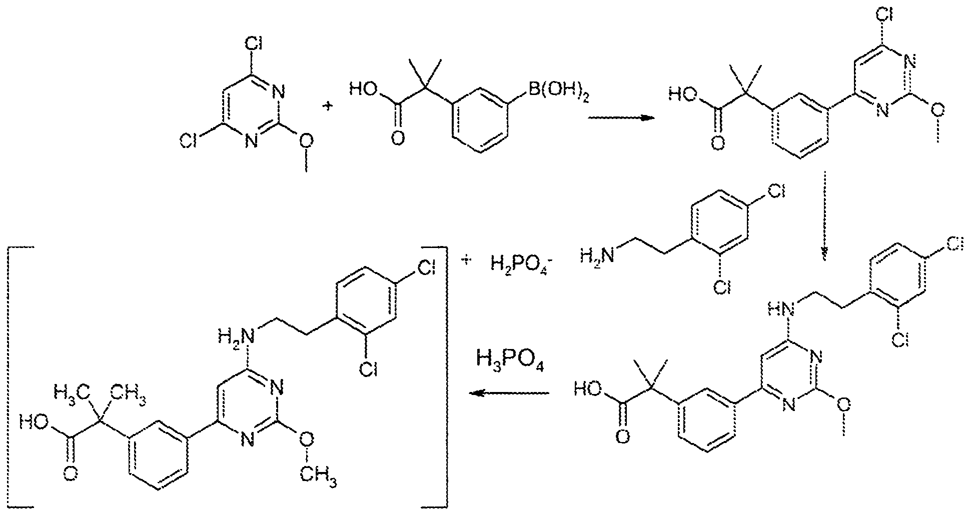

Step 1: To an 1-L reactor are added 4,6-dichloro-2-methoxy-pyrimidine (50.1 g), K₂CO₃ (96.4 g,), Pd(OAc)₂ (48 mg, 0.1 mol%) and PPh₃ (120 mg, 0.2 mol%) in DME/water (100 mL/200 mL). The mixture is heated to 50°C, 3-(1-carboxy-1-methyl-ethyl)-phenyl boronic acid (51.6 g, 94wt% purity) in DME (50 mL)/water (100 mL) is added over 2 h via a metering pump. The reaction is held at 50°C for an additional hour. The mixture is cooled to 25°C and toluene (250 mL) is added. The mixture is stirred for 15 min and the layers are separated. To the aqueous layer is added n-BuOAc (300 mL) and pH is adjusted to witch 4 M aqueous HCl (~ 190 mL). The aqueous layer is extracted with n-BuOAc (2 x 300 mL). The combined organic Savers are treated with TMT (3.2 g) and charcoal (6.4 g) at 70°C for 3 h. The mixture is allowed to cool to any filtered through celite. The filtrate is extracted twice with an aqueous K₂CO₃ solution (31.8 g of K₂CO₃ in 320 mL of water). The combined aqueous layers are used directly in the next step.

Step 2: To the shove aqueous layer is added 2,4-dichlorophenethylamine (43.5 g). The resulting mixture is heated to 95°C for 6 h. The mixture is cooled to 50°C and n-BuOAc (150 mL) is added. The pH is adjusted to ~ 4.94 with an aqueous solution of 4 M HCl (~ 204 mL). The mixture is then cooled to 25°C over 30 min and further cooled to 4°C over 30 min. The mixture is filtered and the wet cake is washed with water (2 x 200 mL) and dried to afford 2-(3-{6-[2-(2,4-dichloro-phenyl)-ethylamino]-2-methoxy-pyrimidin-4-yl}-phenyl)-2-methyl-propionic acid (76.2 g, 97.5%) purity by HPLC), which is further purified by recrystallization from DME/water to provide over 99% purity by HPLC (see FIGURE 1).

### Example 2:

### Dihydrogen phosphate salt or 2-(3-{6-[2-(2,4-dichlorophenyl)-ethylamino]-2-methoxypyrimidin-4-yl}-phenyl)-2-methylpropionic acid

A 1-L jacketed reactor is charged with 2-(3-{6-[2-(2,4-dichloro-phenyl)-ethylamino]-2-methoxy-pyrimidin-4-yl}-phenyl)-2-methylpropionic acid (50.0 g, 0.109 mol) and methanol (500 mL). The mixture is heated to 37.5±2 °C. A 1:10 phosphoric acid:MeOH solution (110 mL) is then added to the mixture over 3 hrs. The mixture is then cooled to 10°C and the temperature is maintained for 0.5 hrs. The solid is collected by filtration and the filter cake is washed with MeOH (120 mL) and dried to give 2-(3-{6-[2-(2,4-dichloro-phenyl)-ethylamino]-2-methoxy-pyrimidin-4-yl}-phenyl)-2-methylpropionic acid phosphoric acid salt (57.9 g, 95.6% yield). Gas Chromatography shows that the product contains 201 ppm residual well below the limit of ICH guideline of 3000 ppm.

The purity of the compound is analyzed by HPLC using the following conditions:

| | | |
|---|---|---|
| Instrument: | Agilent 1100 series HPLC | |
| Column: | Phenomenex Synergi 4µ Hydro-RP, 150 x 4.6mm | |
| Conditions: | Mobile phase: | A: 0.1%TFA in acetonitrile; B: 0.1% TFA in water |
| | Flow rate: | 1.5 mL/min |
| | Detector: | 220 nm |
| | Injection: | 10 µL |
| | Temperature: | 25°C |
| | Run time: | 18 min |
| Gradient: | | |

| Time (min) | Mobile Phase A | Mobile Phase B |
|---|---|---|
| 0 | 30 | 70 |
| 2.0 | 30 | 70 |
| 15.0 | 70 | 30 |
| 18.0 | 90 | 10 |

### Sample preparation:

Dissolve testing sample in water/0.1% TFA in acetonitrile (20/80).

## Claims

1. A process for preparing 2-(3-{6-[2-(2,4-dichloro-phenyl)-ethylamino]-2-methoxy-pyrimidin-4-yl}-phenyl)-2-methyl-propionic acid of formula (A), 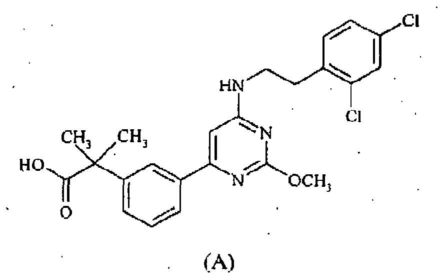 comprising:
(1) a coupling 4,6-dichloro-2-methoxy-pyrimidine with 3-(1-carboxy-1-methyl-ethyl)-phenyl boronic acid in the presence of a Palladium catalyst to provide 2-[3-(6-chloro-2-methoxy-pyrimidin-4-yl)-phenyl]-2-methyl-propionic acid,
(2) coupling 2-[3-(6-chloro-2-methoxy-pyrimidin-4-yl)-phenyl]-2-methyl-propionic acid with 2,4-dichlorophenethylamine to provide 2-(3-{6-[2-(2,4-dichloro-phenyl)-ethylamino]-2-methoxy-pyrimidin-4-yl}-phenyl)-2-methyl-propionic acid.

2. The process according to claim 1, wherein the Palladium catalyst is Pd, PdCl₂, *pddf*, or Pd(OAc)₂.

3. The process according to any of claims 1 to 2, wherein the coupling of 2-[3-(6-chloro-2-methoxy-pyrimidin-4-yl)-phenyl]-2-methyl-propionic acid with 2,4-dichlorophenethylamine is carried out in the presence of PPh₃.

4. The process according to claim 3, wherein the amount of Pd(OAc)₂ is 0.01 mol% to 0.5 mol% and the amount of PPh₃ is 0.02 mol% to 1.0 mol%.

5. The process according to any of claims 1 to 4, wherein the first step coupling reaction is carried out by adding 3-(1-carboxy-1-methyl-ethyl)-phenyl boronic acid to 4.6-dichloro-2-methoxypyrimidine over a period of time of 2-6 hours.

6. The process according to any of claims 1 to 5, wherein the first step coupling reaction is carried out is the presence of a base.

7. The process according to claim 6, wherein the amount of the base is 2 to 4 equivalents.

8. The process according to any of claims 1 to 7, wherein the first step coupling reaction is carried out using 1.0 to 2.0 equivalents of 4,6-dichloro-2-methoxypyrimidine.

9. The process according to any of claims 1 to 8, wherein the first step coupling reaction is carried out in the presence of a solvent system.

10. The process according to any of claims 1 to 9, wherein before coupling 2-[3-(6-chloro-2-methoxy-pyrimidin-4-yl)-phenyl]-2-methyl-propionic acid with 2,4-dichlorophenethylamine, 2-[3-(6-chloro-2-methoxy-pyrimidin-4-yl)-phenyl]-2-methylpropionic acid is purified by steps comprising:
removing excess 4,6-dichloro-2-methoxypyrimidne by a phase separation,
adjusting pH of the phase containing 2-[3-(6-chloro-2-methoxy-pyrimidin-4-yl)-phenyl]-2-methylpropionic acid to 6.5 to 7.5 and
extracting the phase containing 2-[3-(6-chloro-2-methoxy-pyrimidin-4-yl)-phenyl]-2-methylpropionic acid with a water-immiscible organic solvent.

11. The process according to any of claims 1 to 10, wherein the Palladium catalyst is removed from 2-[3-(6-chloro-2-methoxy-pyrimidin-4-yl)-phenyl]-2-methyl-propionic acid before coupling 2-[3-(6-chloro-2-methoxy-pyrimidin-4-yl)-phenyl]-2-methyl-propionic acid with 2,4-dichlorophenethylamine.

12. The process according to claim 11, wherein the Palladium catalyst is removed by treating 2-[3-(6-chloro-2-methoxy-pyrimidin-4-yl)-phenyl]-2-methyl-propionic acid with TMT and charcoal.

13. The process according to claim 12, wherein the Palladium catalyst is removed by treating 2-[3-(6-chloro-2-methoxy-pyrimidin-4-yl)-phenyl]-2-methyl-propionic acid with TMT and charcoal in an organic solvent at 50-70°C.

14. The process according to any of claims 1 to 13, wherein 2-[3-(6-chloro-2-methoxy-pyrimidin-4-yl)-phenyl]-2-methyl-propionic acid is used directly in the second step as a solution.

15. The process according to any of claims 1 to 14, wherein the second step is carried out in water.

16. The process according to any of claims 1 to 15, further comprising purifying 2-(3-{6-[2-(2,4-dichlorophenyl)-ethylamino]-2-methoxypyrimidin-4-yl}-phenyl)-2-methylpropionic acid by recrystallization from an organic solvent.

17. A process according to claim 1 for preparing dihydrogen phosphate salt of 2-(3-{6-[2-(2,4-dichlorophenyl)-ethylamino]-2-methoxypyrimidin-4-yl}-phenyl)-2-methylpropionic acid, further comprising reacting 2-(3- {6-[2-(2,4-dichiorophenyl)-ethylamino]-2-methoxypyrimidin-4-yl}-phenyl)-2-methylpropionic acid with phosphoric acid in methanol.

18. A compound, which is 2-[3-(6-chloro-2-methoxypyrimidin-4-yl)-phenyl]-2-methylpropionic acid, or a salt thereof.

19. The compound according to claim 18, which is 2-[3-(6-chloro-2-methoxypyrimidin-4-yl)-phenyl]-2-methylpropionic acid.

## Patentansprüche

1. Verfahren zur Herstellung von 2-(3-{6-[2-(2,4-Dichlorphenyl)ethylamino]-2-methoxypyrimidin-4-yl}-phenyl)-2-methylpropionsäure der Formel (A), 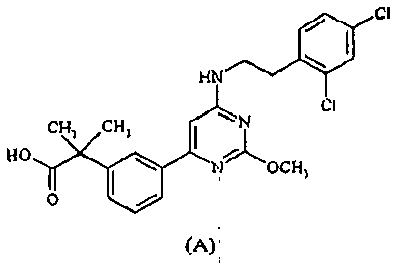 bei dem man:
(1) 4,6-Dichlor-2-methoxypyrimidin in Gegenwart eines Palladiumkatalysators mit 3-(1-Carboxy-1-methylethyl)phenylboronsäure kuppelt, was 2-[3-(6-Chlor-2-methoxypyrimidin-4-yl)phenyl]-2-methylpropionsäure ergibt,
(2) 2-[3-(6-Chlor-2-methoxypyrimidin-4-yl)phenyl]-2-methylpropionsäure mit 2,4-Dichlorphenethylamin kuppelt, was 2-(3-{6-[2-(2,4-Dichlorphenyl)ethylamino]-2-methoxypyrimidin-4-yl}phenyl)-2-methylpropionsäure ergibt.

2. verfahren nach Anspruch 1, bei dem es sich bei dem Palladiumkatalysator um Pd, PdCl₂, *pddf* oder Pd(OAc)₂ handelt.

3. Verfahren nach einem der Ansprüche 1 bis 2, bei dem man die Kupplung von 2-[3-(6-Chlor-2-methoxypyrimidin-4-yl)phenyl]-2-methylpropionsäure mit 2,4-Dichlorphenethylamin in Gegenwart von PPh₃ durchführt.

4. Verfahren nach Anspruch 3, bei dem die Menge an Pd(OAc)₂ 0,01 Mol-% bis 0,5 Mol-% beträgt und die Menge an PPh₃ 0,02 Mol-% bis 1,0 Mol-% beträgt.

5. Verfahren nach einem der Ansprüche 1 bis 4, bei dem man die Kupplungsreaktion des ersten Schritts durch Zugabe von 3-(1-Carboxy-1-methylethyl)-phenylboronsäure zu 4,6-Dichlor-2-methoxypyrimidin über einen Zeitraum von 2-6 Stunden durchführt.

6. Verfahren nach einem der Ansprüche 1 bis 5, bei dem man die Kupplungsreaktion des ersten Schritts in Gegenwart einer Base durchführt.

7. Verfahren nach Anspruch 6, bei dem die Menge der Base 2 bis 4 Äquivalente beträgt.

8. Verfahren nach einem der Ansprüche 1 bis 7, bei dem man die Kupplungsreaktion des ersten Schritts unter Verwendung von 1,0 bis 2,0 Äquivalenten 4,6-Dichlor-2-methoxypyrimidin durchführt.

9. Verfahren nach einem der Ansprüche 1 bis 8, bei dem man die Kupplungsreaktion des ersten Schritts in Gegenwart eines Lösungsmittelsystems durchführt.

10. Verfahren nach einem der Ansprüche 1 bis 9, bei dem man vor der Kupplung von 2-[3-(6-Chlor-2-methoxypyrimidin-4-yl)phenyl]-2-methylpropionsäure mit 2,4-Dichlorphenethylamin die 2-[3-(6-Chlor-2-methoxypyrimidin-4-yl)phenyl]-2-methylpropionsäure durch Schritte reinigt, bei denen man:
überschüssiges 4,6-Dichlor-2-methoxypyrimidin durch Phasentrennung entfernt,
den pH-Wert der 2-[3-(6-Chlor-2-methoxypyrimidin-4-yl)phenyl]-2-methylpropionsäure enthaltenden Phase auf 6,5 bis 7,5 einstellt und
die 2-[3-(6-Chlor-2-methoxypyrimidin-4-yl)-phenyl]-2-methylpropionsäure enthaltende Phase mit einem nicht mit Wasser mischbaren organischen Lösungsmittel extrahiert.

11. Verfahren nach einem der Ansprüche 1 bis 10, bei dem man vor der Kupplung von 2-[3-(6-Chlor-2-methoxypyrimidin-4-yl)phenyl]-2-methylpropionsäure mit 2,4-Dichlorphenethylamin den Palladiumkatalysator aus der 2-[3-(6-Chlor-2-methoxypyrimidin-4-yl)phenyl]-2-methylpropionsäure entfernt.

12. Verfahren nach Anspruch 11, bei dem man den Palladiumkatalysator entfernt, indem man die 2-[3-(6-Chlor-2-methoxypyrimidin-4-yl)phenyl]-2-methylpropionsäure mit TMT und Kohle behandelt.

13. Verfahren nach Anspruch 12, bei dem man den Palladiumkatalysator entfernt, indem man die 2-[3-(6-Chlor-2-methoxypyrimidin-4-yl)phenyl]-2-methylpropionsäure in einem organischen Lösungsmittel bei 50-70°C mit TMT und Kohle behandelt.

14. Verfahren nach einem der Ansprüche 1 bis 13, bei dem man die 2-[3-(6-Chlor-2-methoxypyrimidin-4-yl)phenyl]-2-methylpropionsäure als Lösung direkt im zweiten Schritt verwendet.

15. Verfahren nach einem der Ansprüche 1 bis 14, bei dem man den zweiten Schritt in Wasser durchführt.

16. Verfahren nach einem der Ansprüche 1 bis 15, bei dem man ferner die 2-(3-{6-[2-(2,4-Dichlorphenyl)-ethylamino]-2-methoxypyrimidin-4-yl}phenyl)-2-methylpropionsäure durch Umkristallisation aus einem organischen Lösungsmittel reinigt..

17. Verfahren nach Anspruch 1 zur Herstellung des Dihydrophosphatsalzes von 2-(3-{6-[2-(2,4-Dichlorphenyl)ethylamino]-2-methoxypyrimidin-4-yl}-phenyl)-2-methylpropionsäure, bei dem man ferner 2-(3-{6-[2-(2,4-Dichlorphenyl)ethylamino]-2-methoxypyrimidin-4-yl}phenyl)-2-methylpropionsäure in Methanol mit Phosphorsäure umsetzt.

18. Verbindung, bei der es sich um 2-[3-(6-Chlor-2-methoxypyrimidin-4-yl)phenyl]-2-methylpropionsäure oder ein Salz davon handelt.

19. Verbindung nach Anspruch 18, bei der es sich um 2-[3-(6-Chlor-2-methoxypyrimidin-4-yl)phenyl]-2-methylpropionsäure handelt.

## Revendications

1. Procédé de synthèse de l'acide 2-(3-{6-[2-(2,4-dichloro-phényl)-éthylamino]-2-méthoxy-pyrimidin-4-yl}-phényl)-2-méthyl-propionique de formule (A), 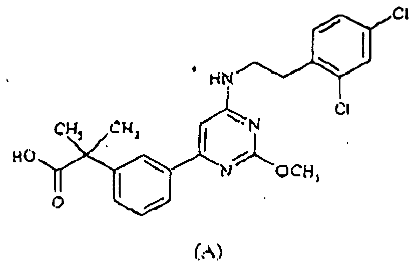 comprenant :
(1) le couplage de la 4,6-dichloro-2-méthoxy-pyrimidine avec l'acide 3-(1-carboxy-1-méthyl-éthyl)-phénylboronique en présence d'un catalyseur au palladium pour obtenir l'acide 2-[3-(6-chloro-2-méthoxy-pyrimidin-4-yl)-phényl]-2-méthyl-propionique,
(2) le couplage de l'acide 2-[3-(6-chloro-2-méthoxy-pyrimidin-4-yl)-phényl]-2-méthyl-propionique avec la 2,4-dichlorophénéthylamine pour obtenir l'acide 2-(3-{6-[2-(2,4-dichloro-phényl)-éthylamino]-2-méthoxy-pyrimidin-4-yl}-phényl)-2-méthyl-propionique.

2. Procédé conforme à la revendication 1, où le catalyseur au palladium est Pd, PdCl₂, *pddf* ou Pd(OAc)₂.

3. Procédé conforme à l'une quelconque des revendications 1 à 2, où le couplage de l'acide 2-[3-(6-chloro-2-méthoxy-pyrimidin-4-yl)-phényl]-2-méthylpropionique avec la 2,4-dichlorophénéthylamine est mis en oeuvre en présence de PPh₃.

4. Procédé conforme à la revendication 3, où la teneur en Pd(OAc)₂ est comprise entre 0,01 % molaire et 0,5 % molaire, et la teneur en PPh₃ est comprise entre 0,02 % molaire et 1,0 % molaire.

5. Procédé conforme à l'une quelconque des revendications 1 à 4, où la réaction de couplage de la première étape est mise en oeuvre en ajoutant de l'acide 3-(1-carboxy-1-méthyl-éthyl)-phénylboronique à de la 4,6-dichloro-2-méthoxypyrimidine sur une durée de 2 à 6 heures.

6. Procédé conforme à l'une quelconque des revendications 1 à 5, où la réaction de couplage de la première étape est mise en oeuvre en présence d'une base.

7. Procédé conforme à la revendication 6, où la quantité de base est de 2 à 4, équivalents.

8. Procédé conforme à l'une quelconque des revendications 1 à 7, où la réaction de couplage de la première étape est mise en oeuvre avec 1,0 à 2,0 équivalents de 4,6-dichloro-2-méthoxypyrimidine.

9. Procédé conforme à l'une quelconque des revendications 1 à 8, où la réaction de couplage de la première étape est mise en oeuvre en présence d'un système solvant.

10. Procédé conforme à l'une quelconque des revendications 1 à 9, où avant le couplage de l'acide 2-[3-(6-chloro-2-méthoxy-pyrimidin-4-yl)-phényl]-2-méthyl-propionique avec la 2,4-dichlorophénéthylamine, l'acide 2-[3-(6-chloro-2-méthoxy-pyrimidin-4-yl)-phényl]-2-méthylpropionique est purifié par des étapes comprenant :
l'élimination de l'excès de 4,6-dichloro-2-méthoxypyrimidine par séparation de phase,
l'ajustement du pH de la phase contenant l'acide 2-[3-(6-chloro-2-méthoxy-pyrimidin-4-yl)-phényl]-2-méthylpropionique entre 6,5 et 7,5, et
l'extraction de la phase contenant l'acide 2-[3-(6-chloro-2-méthoxy-pyrimidin-4-yl)-phényl]-2-méthylpropionique avec un solvant organique immiscible à l'eau.

11. Procédé conforme à l'une quelconque des revendications 1 à 10, où le catalyseur au palladium est séparé de l'acide 2-[3-(6-chloro-2-méthoxy-pyrimidin-4-yl)-phényl]-2-méthyl-propionique avant le couplage de l'acide 2-[3-(6-chloro-2-méthoxy-pyrimidin-4-yl)-phényl]-2--méthylpropionique avec la 2,4-dichlorophénéthylamine.

12. Procédé conforme à la revendication 11, où le catalyseur au palladium est éliminé par traitement de l'acide 2-[3-(6-chloro-2-méthoxy-pyrimidin-4-yl)-phényl]-2-méthyl-propionique par du TMT et du charbon.

13. Procédé conforme à la revendication 12, où le catalyseur au palladium est éliminé par traitement de l'acide 2-[3-(6-chloro-2-méthoxy-pyrimidin-4-yl)-phényl]-2-méthyl-propionique par du TMT et du charbon dans un solvant organique à une température comprise entre 50 et 70°C.

14. Procédé conforme à l'une quelconque des revendications 1 à 13, où l'acide 2-[3-(6-chloro-2-méthoxy-pyrimidin-4-yl)-phényl]-2-méthyl-propionique est employé directement dans la seconde étape sous forme d'une solution.

15. Procédé conforme à l'une quelconque des revendications 1 à 14, où la seconde étape est mise en oeuvre dans l'eau.

16. Procédé conforme à l'une quelconque des revendications 1 à 15, comprenant en outre la purification de l'acide 2-(3-{6-(2-[2,4-dichlorophényl)-éthylamino]-2-méthoxypyridin-4-yl}-phényl)-2-méthylpropionique par recristallisation dans un solvant organique.

17. Procédé conforme à la revendication 1 pour la synthèse du sel de dihydrogénophosphate de l'acide 2-(3-{6-[2-(2,4-dichlorophényl)-éthylamino]-2-méthoxypyrimidin-4-yl}-phényl)-2-méthylpropionique, comprenant en outre la réaction de l'acide 2-(3-{6-[2-(2,4-dichlorophényl)-éthylamino]-2-méthoxypyrimidin-4-yl}-phényl)-2-méthylpropionique avec l'acide phosphorique dans le méthanol.

18. Composé qui est l'acide 2-[3-(6-chloro-2-méthoxypyrimidin-4-yl)-phényl]-2-méthylpropionique ou l'un de ses sels.

19. Composé conforme à la revendication 18, qui est l'acide 2-[3-(6-chloro-2-méthoxypyrimidin-4-yl)-phényl]-2-methylpropionique.
